(19) Europäisches Patentamt
European Patent Office
Office européen des brevets

(11) Publication number: **0 056 249**
**B1**

(12) **EUROPEAN PATENT SPECIFICATION**

(45) Date of publication of patent specification: **26.06.85**

(21) Application number: **82100075.9**

(22) Date of filing: **08.01.82**

(51) Int. Cl.$^4$: **A 61 K 39/00,** C 07 K 7/06, C 07 K 7/08, C 07 K 1/04

(54) **Synthetic antigenic composition and process for making same.**

(30) Priority: **09.01.81 US 223558**
**12.06.81 US 272855**

(43) Date of publication of application:
**21.07.82 Bulletin 82/29**

(45) Publication of the grant of the patent:
**26.06.85 Bulletin 85/26**

(84) Designated Contracting States:
**AT BE CH DE FR GB LI NL SE**

(56) References cited:
**EP-A-0 028 563**
**GB-A-2 045 256**

**CHEMICAL ABSTRACTS, vol. 88, no. 1, 2nd January 1978, page 410, no. 4589p, Columbus Ohio (USA); F. AUDIBERT et al.: "Enhancement of the immune response to influenza vaccines by a synthetic glycopeptide adjuvant (N-acetylmuramyl-L-alanyl-D-isoglutamine)**

(73) Proprietor: **New York Blood Center, Inc.**
**310 East 67 Street**
**New York, New York 10021 (US)**

(72) Inventor: **Hopp, Thomas Patrick**
**548 East 82nd Street**
**New York, N.Y. 10028 (US)**

(74) Representative: **Patentanwaltsbüro Cohausz & Florack**
**Postfach 14 01 47**
**D-4000 Düsseldorf 1 (DE)**

(56) References cited:
**CHEMICAL ABSTRACTS, vol. 92, no. 5, 4th February 1980, page 587, no. 39507v, Columbus Ohio (USA); R. ARNON et al.: "Anti-viral activity induced by synthetic peptides corresponding to regions involved in viral neutralization"**
**CHEMICAL ABSTRACTS, vol. 95, no. 18, 2nd November 1981, page 367, no. 156407k, Columbus Ohio (USA); M. SELA et al.: "From synthetic antigens to synthetic vaccines"**
**NATURE, vol. 289, no. 5798, 12th February 1981, pages 593-594; F. AUDIBERT et al.: "Active antitoxic immunization by a diphtheria toxin synthetic oligopeptide"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 056 249 B1

**Description**

Background of the invention

Field of the invention

This invention relates to a synthetic vaccine and to a process for its preparation. More especially, this invention relates to a method of determining the antigenic or allergenic determinants of protein antigens or allergens and on the basis of the determination of the point of greatest local average hydrophilicity of such protein antigens or allergens, forming a synthetic vaccine containing a designated sequence of six amino acids corresponding to the point of greatest local average hydrophilicity. Thus, the invention relates to the determination of the antigenic or allergenic determinants and on the basis of such information, the formation of synthetic vaccines which provide a protective immunological response or a decrease in allergen sensitivity, as the case may be.

Discussion of prior art

Vaccines have long been known. In the early days vaccines were prepared by the use of active viral components. In more modern days the viral components have been inactivated, and the entire protein has been used to effect an immunological response. More recently, attempts have been made at segregating those proteins of a virus which contain the antigenic or allergenic determinants. Thus, for instance, in the hepatitis vaccine core material of the virus is generally removed, and the vaccine comprises components of the surface of the virus, the vaccine being designated as containing hepatitis B surface antigen. It will be recognized, however, that in the case of a hepatitis vaccine, for example, the entire protein of the surface antigen is present. Much of the polypeptide chain of this protein is inactive insofar as affecting the specificity of the protective immunological response when introduced into a host animal.

The manufacture of such vaccine is extremely expensive, since the proteins containing the antigenic determinants must be obtained from sera or other sources in a laborious and timeconsuming task. Moreover, the preparation of the vaccine must be under strictly controlled conditions which insure that the viral component is completely and totally inactivated. Substantial quantities of sera taken from host animals are required for the realization of a single dose of the viral inactivated vaccine. Many of the components of the vaccine are inactive, but heretofore one has had no other alternative but to use the entire protein, since the molecular portion of the protein responsible for an antigenic or allergenic response was unknown.

Attempts have been made to identify the portions of the protein molecule of an antigen or allergen which is responsible for an immunogenic response. These attempts have involved a study of a structure of the various protein molecules, but no method has heretofore been devised which always provides the researcher with a sequence of amino acids of the protein molecule which is antigenic. It has, therefore, long been desired to provide a means by which the point on a protein molecule which stimulates an immunological response can be reliably determined and predicted so that on the basis of such knowledge a synthetic vaccine containing a sequence of amino acids corresponding to such portion of the protein molecule can be synthetically formed. Heretofore, synthetic vaccines which provide a protective immunological response or decrease in allergen sensitivity have not been provided.

It is an object of this invention, therefore, to provide the researcher with a tool by which the sequence of amino acids corresponding to the antigen portion of a given protein antigen can be reliably predicted. It is a further object of this invention to provide a process for the synthesis of a synthetic vaccine containing moieties corresponding to such sequence of amino acids. More especially, it is an object of this invention to provide a synthetic vaccine containing such sequence of amino acids.

It is a particular object of this invention to provide a synthetic vaccine which is free of amino acid sequences corresponding to the entire amino acid sequence of the protein. More especially, it is an object of this invention to provide a synthetic vaccine which is free of biologically produced materials, free of active or inactive viral residues, free of lipids and free of DNA.

It is a further object of this invention to provide a synthetic vaccine which can be formed without use of natural sera and which is free of contaminating serum proteins.

Summary of the invention

In accordance with this invention there is provided a synthetic vaccine comprising a physiologically acceptable carrier in which is disposed a synthetic peptide residue containing a sequence of six amino acids corresponding to the sequence of such amino acids in a protein antigen or allergen where the greatest local average hydrophilicity of the antigen or allergen is found, said local hydrophilicity of said protein antigen or allergen defined by:

A. assigning relative hydrophilicity values to the amino acids of the protein antigen or allergen in accordance with relative relationship of such amino acids as shown in the table below:

TABLE 1

| Amino acid | Hydrophilicity value |
|---|---|
| Arginine | 3.0 |
| Aspartic Acid | 3.0±1 |
| Glutamic Acid | 3.0±1 |
| Lysine | 3.0 |
| Serine | 0.3 |
| Asparagine | 0.2 |
| Glutamine | 0.2 |
| Glycine | 0.0 |
| Proline | −0.5±1 |
| Threonine | −0.4 |
| Alanine | −0.5 |
| Histidine | −0.5 |
| Cysteine | −1.0 |
| Methionine | −1.3 |
| Valine | −1.5 |
| Isoleucine | −1.8 |
| Leucine | −1.8 |
| Tyrosine | −2.3 |
| Phenylalanine | −2.5 |
| Tryptophan | −3.4 |

B. Determining the repetitive local average of hydrophilicity values at a plurality of points along the amino acid sequence:

C. determining from such local points of repetitive averages the points of greatest local average hydrophilicity;

said composition characterized by evoking a protective immunological response or by stimulation of antibody formation or decreased sensitivity to allergen when introduced into a host animal in the absence of the entire amino acid sequence of the protein antigen or allergen.

At the heart of the invention there is the determination of the sequence of six amino acids which are critical to the production of the immunological response. In accordance with the invention, this is done with the foreknowledge of the amino acid sequence of an antigen or allergen. If the same is unknown, then the amino acid sequence of the entire protein must first be determined. This can be determined by known but laborious means.

Given the amino acid sequence of the entire protein antigen or allergen, the next objective is to determine the point along said molecule where there is greatest local average hydrophilicity. This is initially done by assigning relative hydrophilicity values in accordance with the table above to each amino acid in the protein. Thereafter, those values are repetitively averaged along the length of the protein. While such method is partially successful (working for some proteins, but not others) when averaging groups range in size from four to ten successively connected amino acids, it is preferred that in determining such local averages the hydrophilicity values of five to seven linearly connected amino acids be employed, especially six such amino acids. At a plurality of points along the amino acid chain of the protein, the local averages are determined (moving average, increment of one).

Once the repetitive local averages of the specific hydrophilicity values are determined, the precise

point of greatest hydrophilicity can be easily located by inspection or determined graphically or otherwise. It has been discovered that the six amino acids providing the greatest local average hydrophilicity are the sequence of six amino acids which are critical to the production of the immunological response. Stated differently, it has been found that this sequence of six amino acids is present in an epitope of the protein, i.e. the sequence of amino acids recognized by and bound by an antibody with immunological specificity. Such epitope, is hereinafter designated as the "H-epitope" as it is the epitope of greatest local average hydrophilicity.

With this realization of the precise sequence of amino acids which accounts for H-epitope of a given protein antigen or allergen, one can form a synthetic vaccine in any number of ways.

The synthetic vaccine is prepared either by chemically synthesizing a chain of amino acids corresponding to the sequence of amino acids of the H-epitope or the H-epitope is obtained from a protein containing the same by selective lysis such as by splitting the protein by the use of enzymes. The amino acid chain containing the H-epitope so obtained either synthetically or from naturally occurring protein is thereafter bonded on a physiologically acceptable carrier, which can be a polymer of itself, and the resultant composition is thereafter diluted with a physiologically acceptable medium. The composition is then ready for introduction into a host animal.

It will be realized that the process of the invention is useful in the formation of synthetic vaccines of known and unknown, identified or unidentified, protein antigens or allergens, since the focus is upon that portion of the protein molecule which provides the H-epitope. Thus, the synthetic vaccine of the invention can contain H-epitopes of single or multiple known or unknown protein antigens or allegens. The synthetic vaccine can contain a plurality of H-epitopes of a single antigen or can contain a single H-epitope of a first antigen and a H-epitope of a second antigen or allergen. The synthetic vaccine can contain one or more H-epitopes of an antigen or allergen alone or in combination with one or more H-epitopes of a second antigen or allergen. In fact, the synthetic vaccine can contain as many epitopes corresponding to said sequence of six amino acids of greatest local average hydrophilicity as desired, and said epitopes can correspond to the sequence of six amino acids from a wide variety of antigens or allergens. The vaccine contains at least one H-epitope. This H-epitope can be co-present with other epitopes of the same or different antigens which are not H-epitopes; i.e. do not correspond to the point of greatest local average hydrophilicity of the antigen or allergen.

The process of the invention is useful in the formation of synthetic vaccines from antigens whose amino sequence has not heretofore been reported. The art well knows how to determine the amino acid sequence of a protein antigen or allergen. It remains, therefore, a simple matter in accordance with the invention to determine the H-epitope.

The synthetic vaccine can have H-epitopes of any protein antigen or allergen. The vaccine of the following protein antigens or allergens are particularly contemplated. Hepatitis B surface antigen, histocompatibility antigens, influenza hemagglutinin, fowl plague virus hemagglutinin, rag weed allergens Ra3 and Ra5 and the antigens of the following viruses: vaccinia, Epstein-Barr virus, polio, rubella, cytomegalovirus, small pox, herpes, simplex types I and II, yellow fever, and many others.

It can also alternatively or additionally have an H-epitope of a protein of any of the following parasites: organisms carrying malaria (P. Falciporum, P. Ovace, etc.), Schistosomiasis, Onchocerca Volvulus and other filiarial parasites, Trypanosomes, Leishmania, Chagas disease, amoebiasis, hookworm, and the like. In addition, vaccines of the following bacteria are especially contemplated: leprosy, tuberculosis, syphilis, gonorrhea and the like.

Vaccines of the following viruses can be made by the process of the invention: Infectious ectromelia virus, Fowlpox virus, Herpes simplex virus, Infectious bovine rhinotracheitis virus, Equine rhinopneumonitis (equine abortion) virus, Malignant catarrh virus of cattle, Feline rhinotracheitis virus, Canine herpesvirus, Epstein-Barr virus (ass. with infectious mononucleosis and Burkitt lymphoma), Marek's disease virus, Sheep pulmonary adenomatosis (Jaagziekte virus, Cytomegalo-viruses, Adenovirus group, Human papilloma virus, Feline panleucopaenia virus, Mink enteritis virus, African horse sickness virus (9 serotypes), Blue tongue virus (12 serotypes, Infectious pancreatic necrosis virus of trout, Fowl sarcoma virus (various strains). Avian leukosis virus, visceral, Avian leukosis virus, erythroblastic, Avian leukosis virus, myeloblastic, Osteopetrosis virus, Newcastle disease virus, Parainfluenza virus 1, Parainfluenza virus 2, Parainfluenza virus 3, Parainfluenza virus 4, Mumps virus, Turkey virus, CANADA/58 Canine distemper virus, Measles virus, Respiratory syncytial virus, Myxovirus, Type A viruses such as Human influenza viruses, e.g. Ao/PR8/34, A1/CAM/46, and A2/Singapore/1/57; Fowl plague virus: Type B viruses, e.g. BLee/40; Rabies virus; Eastern equinine encephalitis virus; Venezuelan equine encephalitis virus; Western equine encephalitis virus; Yellow fever virus; Dengue type 1 virus (=type 6), Dengue type 2 virus (=type 5); Dengue type 3 virus; Dengue type 4 virus; Japanese encephalitis virus, Kyasanur Forest virus; Louping ill virus; Murray valley encephalitis virus; Omsk haemorrhagic fever virus (types 1 and 11); St. Louis encephalities virus; Human rhinoviruses, Foot-and-mouth disease virus; Poliovirus type 1; Enterovirus Polio 2; Enterovirus Polio 3; Avian infectious bronchitis virus; Human respiratory virus; Transmissible gastro-enteritis virus of swine; Lymphocytic choriomeningitis virus; Lassa virus; Machupo virus; Pichinde virus; Tacaribe virus; Papillomavirus.

Similarly, the synthetic vaccine can have an H-epitope of any protein allergen such as the rag weed allergens.

It is to be understood that the foregoing lists are not all-inclusive, but simply exemplary, since the heart of the invention resides in reliably and confidently predicting and determining the H-epitope.

In forming a synthetic vaccine according to the invention, it is preferred to insure that the epitope has the steric configuration to be recognized by any antibody; that the given sequence of 6 amino acids have bonded thereto as part of the amino acid chain at least three amino acids on either side thereof, these three adjacent amino acids serving as auxiliary acids to insure the stabilization of the epitope so that it is readily recognized by and neutralized by an antibody.

In one of its simplest forms, the invention comprises a physiologically acceptable carrier on which is bonded a synthetic peptide residue of the designated epitope. This synthetic peptide residue has a chain length of minimally six amino acids, preferably twelve amino acids (considering the three amino acids on either side thereof) and can contain an infinitely long chain of amino acids or their components, which can be characterized by the presence of other epitopes of the same or different antigen or allergen. Where it is free of such additional chain or without such additional epitopes, it generally does not have an amino acid chain exceeding 50 amino acids. Where a short chain is desired containing the desired epitope, it preferably does not have an amino acid chain length greater than 40, more especially not greater than 30 and more particularly not greater than 20 amino acids.

Where, however, the epitope is part of a long chain, such as when there are more than one epitopes on the chain, the chain can contain residues of any of the following moieties: segments of polyamino acid, polysaccharides, polyamides, vinyl polymers, ester polymers, polyacetals, polyolefins, polyphenyl sulfide, polycarbonates as well as bivalent organo radicals, including bivalent alkylene and other saturated or unsaturated organo e.g. hydrocarbon radicals. These residues can have molecular weights of up to 1.000.000, preferably between 10.000 and 100.000, the molecular weight being determined by ultracentrifugation. If the chain comprises an amino acid chain, the chain preferably comprises no more than 2.000 amino acids, excluding amino acids associated with an epitope.

It will be realized that a chain containing the basic sequence of the H-epitope can contain a vaccine adjuvant. Such vaccine adjuvants include muramyl dipeptide and analogs which can be covalently bonded.

Alternatively, the vaccine can comprise a chain of amino acids containing one or more H-epitopes together with other chains of amino acids forming epitopes of the same antigen or allergen or of different antigens or allergens. These additional chains can be of the same or different chain length. The chains which can contain epitopes can be interconnected with one another such as by crosslinking or by being bonded directly thereto in the form of a branched chain, or the respective chains are bonded to a central "carrier". Where there is a plurality of epitope containing chains, a given epitope chain can contain a chain adjacent the epitope of molecular weight up to 1.000.000, determined in accordance with the foregoing method, which chain can serve as a bridge to other epitopes, it being realized that these other epitopes can be epitopes of the same allergen or antigen as the epitope in the chain, the same epitope as the epitope of the antigen or allergen in another chain, or can be a third, fourth or fifth etc., epitope, as the case may be. It is contemplated that the vaccine contain a plurality of the same or different epitopes. In particular, it is contemplated that a vaccine contain between 1 and 1.000 epitopes, of the same antigen or allergen per covalent unit. It can also have present in addition thereto between 1 and 1.000 epitopes per covalent unit of a different antigen or allergen or plurality of different antigens or allergens, all as desired.

The H-epitope requires proper presentation in order to elicit an immune response. To this end, a carrier is provided for such H-epitopes. The "carrier" is simply a physiologically acceptable mass to which the H-epitope is attached. A carrier can comprise simply a chain of amino acids or other moieties and to that end it is specifically contemplated to use as a carrier a dimer, oligomer, or higher molecular weight polymer of sequences containing the six amino acids. In other words, having determined which sequence of six amino acids form the H-epotope, these amino acids can be formed from naturally available materials or synthetically and can be polymerized to build up a chain of two or more repeating units so that repeating sequences serves both as "carrier" and H-epitopes. Stated differently, an independent carrier is not required. It is preferred that alternative carriers comprise some substance, animal, vegetable or mineral, which is physiologically acceptable and functions to present the H-epitope so that it is recognized by the immune system of a host and stimulates a satisfactory immunological response. Thus, a wide variety of carriers are contemplated, and these include materials which are inert, which have biological activity and/or promote an immunological response. For instance, proteins can be used as carriers and there is included within such subclass hemoglobin, human serum proteins, tetanus toxoid.

Polysaccharides are also contemplated as carriers and these include especially those of molecular weight 10.000 to 1.000.000, including in particular starches, dextran, agarose, ficoll or its carboxy methyl derivative and carboxy methyl cellulose.

Polyamino acids are also contemplated for use as carriers and these polyamino acids include, among others, polylysine, polyalanyl polylysine, polyglutamic acid, polyaspartic acids and poly ($C_2$—$C_{10}$) amino acids.

Vaccines can be used as carriers for the epitopes provided by the invention. In other words, the synthetic or naturally derived peptide residues provided by the invention can themselves be attached to other vaccines including vaccines for measles, influenza, smallpox, polio, diphtheria, pneumonococci, meningococci, as well as the vaccines of the virus and organisms mentioned above.

The carrier can conveniently be a living organism such as bacteria.

Organic polymers can be used as carriers, and these polymers include polymers and copolymers of amines, amides, olefins, vinyls, esters, acetal, polyamides, carbonates, ethers, phenylene sulfides, silicones, urea formaldehyde condensation products, phenol formaldehyde condensation products, urethanes, melamine formaldehydes, epoxy resins, acrylic resins, allyl resins, and the like. Generally speaking, the molecular weight of these polymers will vary dramatically. The polymers can have from two repeating units up to several thousand e.g. two thousand repeating units. Of course, the number of repeating units will be consistent with the use of the vaccine in a host animal. Generally speaking, such polymers will have a lower molecular weight, say between 10.000 and 100.000, determined in accordance with the procedure set forth above.

Inorganic polymers can also be employed. These inorganic polymers can be inorganic polymers containing organo moieties. In particular, silicates can be used as carriers. It is preferred that the carrier be one which is an immunological adjuvant. In such cases, it is particularly contemplated that the adjuvant be any one of the following: muramyl dipeptide or its analogs.

The carrier can also be the residue of a crosslinking agent employed to interconnect a plurality of epitope-containing chains. The crosslinking agent can be one which interconnects the chains at a point containing the sequence of six amino acids. Alternatively, the crosslinking agent can interconnect a plurality of chains at a point other than where the epitope is formed. Crosslinking agents which are contemplated include crosslinking agents which have as their functional group an aldehyde, carboxyl, amine, amido, imido or azidophenyl, group. In particular, there is contemplated the use of butyraldehyde as a crosslinking agent, a divalent imido ester or a carbodiimide. Particularly contemplated divalent imido esters are those of the formula

$$\begin{array}{l} R—O—C{=}N\ H_2{+} \\ \qquad\quad | \\ \qquad\ (CH_2)m \\ \qquad\quad | \\ R—O—C{=}N\ H_2{+} \end{array}$$

wherein m is 1 to 13 and R is an alkyl group of 1 to 4 carbon atoms.

Particularly contemplated carbodiimides for use as crosslinking agents include cyclohexylcarbodiimide, ethyldimethylaminopropyl, carbodiimide, N-ethylmorpholino cyclohexyl carbodiimide, diisopropyl carbodiimide.

It should be understood that the vaccine of the invention can be in admixture with other proteins and these proteins include the proteins of known antigens or allergens. Thus, when it is stated herein that the vaccine is characterized by the absence of an amino acid sequence corresponding to the entire protein antigen or allergen it is meant that notwithstanding the absence of the amino acid sequence of the entire protein antigen or allergen, the composition functions as a vaccine, i.e. provides protective immunization by formation of antibodies in the case of an antigen or a lessoning of allergic sensitivity in the case of an allergen.

The composition of the invention is also useful for purposes other than as a vaccine. It is known, for instance, that certain patients suffering from hemophilia contain within their system an antibody to Factor VIII, Factor VIII being a substance which promotes clotting. It has long been an object to bind the Factor VIII antibody so that it, in turn, cannot interfere with any factor VIII which might be present in the blood stream. By determining the amino acid sequence of the Factor VIII protein, a synthetic antigenic composition can be prepared by the techniques described herein in which antigen compositions have H-epitopes corresponding to the anti-Factor VIII antibody. Such synthetic antigen compositions can be mono-specific to the anti-Factor VIII antibody. When introduced into the host hemophiliac, the H-epitopes in the synthetic antigenic composition are recognized by the anti-Factor VIII antibody with the result that they combine leaving the Factor VIII in the bloodstream free of the anti-Factor VIII antibody.

Description of specific embodiments

In the determination of the sequence of six amino acids which provide the H-epitope, it is preferred that more respective values than those set forth in the table below be assigned to respective amino acids in the protein antigen or allergen. Thus, there is set forth in the table below the broad, preferred and most preferred ranges to be assigned for the determination of six amino acids providing greates local average hydrophilicity.

TABLE 2

| Amino acid | Hydrophilicity value Broad | Preferred | Most preferred |
|---|---|---|---|
| Arginine | 3.0 | 3.0 | 3.0 |
| Aspartic Acid | 3.0±1 | 3.0±5 | 3.0 |
| Glutamic Acid | 3.0±1 | 3.0±5 | 3.0 |
| Lysine | 3.0 | 3.0 | 3.0 |
| Serine | 0.3 | 0.3 | 0.3 |
| Asparagine | 0.2 | 0.2 | 0.2 |
| Glutamine | 0.2 | 0.2 | 0.2 |
| Glycine | 0.0 | 0.0 | 0.0 |
| Proline | −.5±1 | 0.0±5 | 0.0 |
| Threonine | −0.4 | −0.4 | −0.4 |
| Alanine | −0.5 | −0.5 | −0.5 |
| Histidine | −0.5 | −0.5 | −0.5 |
| Cysteine | −1.0 | −1.0 | −1.0 |
| Methionine | −1.3 | −1.3 | −1.3 |
| Valine | −1.5 | −1.5 | −1.5 |
| Isoleucine | −1.8 | −1.8 | −1.8 |
| Leucine | −1.8 | −1.8 | −1.8 |
| Tyrosine | −2.3 | −2.3 | −2.3 |
| Phenylalanine | −2.5 | −2.5 | −2.5 |
| Tryptophan | −3.4 | −3.4 | −3.4 |

It will be recognized that these values are relative. By multiplying these values with a factor, one can obtain another set of values which can be similarly used and provide the same prediction and determination. The important concept is that the respective amino acids have the relative relationship as set forth in the table above. These aribtrary values are established for the purpose of providing a convenient means whereby the portion of a long chain protein molecule of highest hydrophilic characteristic is identified. When that is determined, the realisation of the six amino acids accounting for that hydrophilicity peak is easily determined.

Thus, the procedure of the invention can be employed to determine the sequence of six amino acids of numerous unrelated antigens which provide the greatest hydrophilicity.

Specifically, the hepatitis B surface antigen has been studied to determine the sequence of six amino acids which determine the H-epitope. The sequence of amino acids for such antigen is as follows: Lys Pro Thr Asp Gly Asn (which correspond to amino acids 141—146 of heptatitis B surface antigen protein) Similarly, the sequence of amino acids for the human histocompatibility antigen HLA-B7 which determine the H-epitope is: Pro Arg Glu Glu Pro Arg (which correspond to amino acids 43—48 of the protein).

Similarly, the sequence of amino acids for the influenza hemagglutinin antigen (X31 strain) which determine the H-epitope is: Val Glu Arg Ser Lys Ala (which correspond to amino acids 105—110 of the protein).

The H-epitope for the A/memphis/102/72 strain of influenza hemagglutinin: Lys Arg Gly Pro Asp Ser, corresponding to amino acids 140 to 145 of the protein.

The H-epitopes for two other strains of influenza hemagglutinin, A/Eng/878/69 and A/NT/60/68/29c, are identical to the H-epitope of A/memphis/102/72 as stated above.

The H-epitopes of the A/NT/60/68 and A/Qu/7/70 strains of hemagglutinin are identical and comprise the following amino acids: Arg Asn Val Pro Glu Lys corresponding to amino acids 321—326 of the proteins.

The H-epitope for the neuraminidase protein of the A/PR/8/34 strain of influenza is Arg Gly Arg Pro Lys Glu Lys, corresponding to amino acids 413 to 419 of the protein. This epitope contains seven amino acids because it comprises two adjacent and overlapping H-epitopes of equal hydrophilicity, as is the case for the Japan strain hemagglutinin already described (in the original manuscript).

The H-epitope for the diphtheria toxin fragment A is: Glu Thr Arg Gly Lys Arg, corresponding to amino acids 168 and 173 of the protein.

The H-epitope for the avian sarcoma virus gp 37 protein is: Leu Arg Glu Ile Glu Arg Leu, corresponding to amino acids 37 to 43 of the protein (again, two adjacent and overlapping H-epitopes yielding a seven amino acid sequence).

The H-epitope for the avian sarcoma virus src gene Protein is: Lys Ser Lys Pro Lys Asp, corresponding to amino acids 5 to 10 of the protein.

The H-epitope for the E3/16 protein (external portion) of the adenovirus type 2 strain is: Lys Asp Lys Ile Gly Lys, corresponding to amino acids 40 to 45 of the protein.

The H-epitope for the Simian virus 40 VP1 protein is: Asp Asp Ser Pro Asp Lys Glu, corresponding to amino acids 77 to 83 of the protein (two adjacent and overlapping H-epitopes).

The H-epitope for the available sequence of the fiber protein of adenovirus type 2 (N-terminal 80%) is: Asn Lys Asn Asp Asp Lys, corresponding to amino acids 393 to 398 of the protein.

The H-epitope of the Sinbis virus membrane glycoprotein E1 is: Ser Asp Arg Glu Gly Gln, corresponding to amino acids 322 to 327.

The H-epitope of the Sindbis virus membrane glycoprotein E2 corresponds to the following amino acid chain: Asp Glu Ala Asp Asp Asn, corresponding to amino acids 36 to 41.

The H-epitope for the Sinbis virus membrane glycoprotein E3 corresponds to amino acids 27 to 32 and has the following sequence: Thr Arg Glu Pro Ser Arg.

The H-epitope for the foot and mouth disease virus capsid protein VP1 corresponds to amino acids 179 to 184 and has the following amino acid sequence: Arg Met Lys Arg Al Glu.

There are two sequences of amino acids for the influenza hemagglutinin antigen (Japan strain) which determine H-epitopes of equivalent hydrophilicity i.e. they provide identical local average hydrophilicity. They are Glu Lys Glu Asn Pro Arg (correspond to amino acids 96—101) and Lys Glu Asn Pro Arg Asp (correspond to amino acids 97—102). Similarly, the sequence of amino acids for the influenza hemagglutinin antigen (Victoria A strain) which determine the H-epitope is: Asn Asp Asn Ser Asp Lys (corresponding to amino acids 188—193).

Similarly, there are two sequences of amino acids for the Fowl Plague virus hemagglutinin antigen which determine H-epitopes of identical local average hydrophilicity. They are: Glu Arg Arg Glu Gly Asn (corresponding to amino acids 97—102) and Ar Arg Glu Gly Asn Asp (corresponding to amino acid 98—103).

Similarly, the sequence of amino acids for the human chorionic Gonadotropin B subunit antigen which determine the H-epitope is: Arg Arg Ser Thr Thr Asp corresponding to amino acids 94—99.

Similarly, the sequence of amino acids for the Human-Beta-2 microglobulin antigen which determines the H-epitope is: Pro Thr Glu Lys Asp Glu which corresponds to amino acids 73—78.

Similarly, the sequence of amino acids for the human Myelin basic protein antigen which determines the H-epitope is: Gly Arg Asp Ser Arg Ser corresponding to amino acids 159—164.

Similarly, the sequence of amino acids for the Cholera Toxin B-chain antigen which determines the H-epitopes is: Glu Ala Lys Val Glu Lys corresponding to amino acids 79—84.

Another hepatitis B surface antigen has been studied to determine its sequence of six amino acids which determine the H-epitope. Its sequence is: Lys Pro Ser Asp Gly Asn corresponding to amino acid 141—146.

The sequence of amino acid for the E Coli Heat Labile Toxin which determine the H-epitope is Glu Arg Met Lys Asp Thr corresponding to amino acids 66—71.

The sequence of amino acids for the E Coli Heat Stabile Toxin provides two identical H-epitopes whose amino acid sequence is Asp Ser Ser Lys Glu Lys and Ser Glu Lys Lys Ser Glu corresponding to amino acid 26—31 and 46—51, respectively.

The ragweed allergen Ra3 has an H-epitope whose amino acid sequence is Cys Thr Lys Asp Gln Lys corresponding to amino acid 88—93.

The ragweek allergen Ra5 has an H-epitope whose amino acid sequence is Ser Lys Lys Cys Gly Lys corresponding to amino acids 40—45.

The streptococcial M protein (strain 24) has two identical H-epitopes whose amino acid sequence are Arg Lys Ala Asp Leu Glu and Lys Ala Asp Leu Glu Lys corresponding to amino acids 58—63 and 59—64.

The trypanosoma brucei variant surface glycoprotein 117 has an H-epitope whose amino acid sequence is Lys Als Lys Glu Lys Gly corresponding to amino acids 50—55.

In preparing vaccines according to the invention, it is preferred to attach to the six amino acids which define the H-epitope at least three amino acids on either side thereof. These three amino acids can be the

same amino acids in the same sequences as they occur in the natural protein. However, other amino acids can also be used. For instance, in the hepatitis Bs vaccine the amino acid sequence can be Aba Aba Thr Lys Pro Thr Asp Gly Asn Aba Thr Aba (Aba residues have replaced Cys residues). The synthetic vaccines are prepared as follows:

1. Chemical Synthesis: The Merrifield solid phase procedure is used to build up the appropriate sequence of L-amino acids from the carboxyl terminal amino acid to the amino terminal amino acid. Starting with the appropriate carboxyl terminal amino acid attached to the polystyrene (or other appropriate) resin via chemical linkage of a chloromethyl group, benzhydrylamine group, or other reactive group of the resin, amino acids are added one by one using the following procedure for each:

a) Peptidyl resin is washed with methylene chloride
b) neutralized by mixing for 10 min. at room temperature with 5% (v/v) diisopropylethylamine (or other hindered base) in methylene chloride
c) washed with methylene chloride
d) An amount of amino acid equal to six times the molar amount of the growing peptide chain is activated by combining it with one-half as many moles of a carbodiimide (e.g. dicyclohexylcarbodiimide, diisopropylcarbodiimide) for 10 minutes at 0°C to form the symmetric anhydride of the amino acid. The amino acid used should be provided originally as the N-α-butyl-oxycarbonyl derivative with side chains protected with benzylesters (aspartic and glutarmic acids) benzyl ethers (serine, threonine cysteine, tyrosine), benzyl oxycarbonyl groups (lysine) or other protecting groups commonly used in peptide synthesis.
e) the activated amino acid is reacted with the peptidyl resin for 2 hours at room temperature, resulting in addition of the new amino acid to the end of the growing peptide chain.
f) The resin is washed with methylene chloride
g) The N-α-(butyloxycarbonyl group is removed from the most recently added amino acid by reacting with 30% (v/v) trifluoroacetic acid in methylene chloride for 30 minutes at room temperature.
h) The resin is washed with methylene chloride.
i) Steps a through h are repeated until the required peptide sequence has been constructed.

The peptide is then removed from the resin, and simultaneously the side-chain protecting groups are removed, by reacting with anhydrous hydrofluoric acid containing 10% (v/v of anisole). Subsequently, the peptide can be purified by gel filtration, ion exchange or high pressure liquid chromatography, or other suitable means.

In some cases, chemical synthesis can be carried out without the solid phase resin, in which case the synthetic reactions are performed entirely in solution. The reactions, and the final product, are otherwise essentially identical.

2. Isolation from natural sources: If sufficient quantities of the whole protein antigen are available, a limited portion of the molecule, bearing the H-epitope, may be excised by any of the following procedures:

a) Digestion of the protein by proteolytic enzymes, especially those enzymes whose substrate specifically result in cleavage of the protein at sites immediately adjacent to the H-epitope bearing sequence.
b) Cleavage of the protein by chemical means. Particular bonds between amino acids can be cleaved by reaction with specific reagents. Examples include: bonds involving methionine are cleaved by cyanogen bromide; asparaginyl glycine bonds are cleaved by hydroxylamine; disulfide bonds between two systeine residues are cleaved by reduction (e.g. with dithiothreitol).
c) A combination of proteolytic and chemical changes.

It should also be possible to clone a small portion of the DNA that codes for the H-epitope bearing peptide, resulting in the production of the peptide by bacteria.

The biologically derived H-epitope bearing peptide, once produced, may be purified by gel filtration, ion exchange or high pressure liquid chromatography or other suitable means.

Analogously, one can form chains containing a plurality of H-epitopes of the same or different antigens or allergens by the following technique: An aqueous solution of the epitope bearing peptide or peptides is mixed with a water-soluble carbodiimide (e.g. ethyldimethylaminopropylcarbodiimide). This results in polymerization of the peptide(s); depending on the use of the side chain blocking groups mentioned above, either straight chain or branched polymers of the epitope bearing peptide can be made.

If desired the epitope containing chain employed in the vaccine of the invention can have bonded thereto a chain of any the following moieties: polypeptide, polyaminoacid, polysaccharide, polyamide, polyacrylamide which can serve as a stabilizing chain or as a bridge between epitopes. Such chains are available commercially or, in the case of polyamino acids, are formed by a process which comprises: mixing a solution of epitope bearing peptide with a solution of the N-carboxylanhydride of the amino acid and allowing a base-catalyzed polymerization to occur, which is initiated by the amine groups of the peptide.

The disposition of a chain or chains on a "carrier" is effected as follows:

1. Protein Carrier: The protein and the H epitope bearing peptide are dissolved together in water or

other suitable solvent, and covalently linked via amide bonds formed through the action of a carbodiimide. The resulting product may contain one or more copies of the peptide per protein monomer.

2. Polysaccharide Carriers; Oligosaccharide carriers should have molecular weights in the range 1,000 to 1,000,000. In order to covalently link these to H epitope peptides, suitable functional groups must first be attached to them. Carboxyl groups may be introduced by reacting with iodoacetic acid to yield carboxymethylated polysaccharides, or by reacting with carbonyldiimidazole to yield activated carbonyl esters. Carboxymethyl polysaccharides are coupled to the peptide by a carbodiimide reaction, while the activated carbonyl esters react spontaneously with peptides. Multiple copies of the H epitope bearing peptide should be attached to each oligosaccharide unit.

3. Polyamino Acid Carriers: These carriers should have molecular weights in the range 1,000 to 1,000,000. Polylysine and polyornithine have primary amino groups on their side chains; polyaspartic acid and polyglutamic acid have carboxyl groups. Peptides may be coupled to these via amide bonds using the carbodiimide reaction. Another carrier that provides amino groups for coupling is polysine to which polyalanine has been attached to the side chains of the lysine residues. The H epitope bearing peptide may be attached to the ends of the polyalanine chains, also by a carbodiimide reaction. Multiple copies of the H epitope bearing peptide should be attached to each oligopeptide unit.

The respective epitope containing chains can be linked to one another by a cross-linking agent. Generally speaking, the cross-linking agent can be any one of a type identified above. Crosslinking is effected by reacting the epitope containing peptide residue with the cross-linking agent as follows:

Reaction with glutaraldehyde or a bis-imidate (e.g. dimethylsuberimidate) in aqueous solution results in polymerization of the epitope bearing peptide, with the cross-linking reagent forming covalent bridges between peptide monomers. By the procedure of the invention there is realized a vaccine which is characterized by the absence of an amino acid sequence of the entire protein antigen or allergen. For instance, in the case of a hepatitis B vaccine, the vaccine is free of other peptide sequences of the hepatitis B surface antigen protein, or other proteins found in the virion. Vaccines can be synthesized which are free of biologically produced components, free of virial components whether they be active or inactive, free of antibodies, free of dioxyribonuclaic acid (DNA) and free of lipids, and are therefore likely to be substantially free from undesirable side effects commonly found with other vaccines (unintentional infection with virus, allergic reactions, fevers, etc.).

The synthetic vaccines are characterized by exceptional specificity and evoke an unusual and special response when introduced into a host animal. Whereas a vaccine made of natural material and introduced into a host animal usually evokes an immunological response by the creation of antibodies specific to a number of distinct epitipes present on the antigens found in the vaccine, when the vaccine of the present invention is introduced into a host animal, it causes the formation of antibodies which are mono-specific, i.e. are specific to the single antigenic site on the vaccine. Thus, the vaccines of the present invention can be employed to form immune globulin comprising a monospecific antibody. These monospecific antibodies may be produced in animals, to serve as a source for diagnostic immunoglobulin to be used in serological testing, for example in identifying strain types of pathogenic organisms isolated from infected individuals. In the preparation of a vaccine the concentration of the same in the physiologically acceptable medium will vary depending on the number and types of H epitopes contained therein. Generally speaking, the active component of the vaccine can be present in a concentration which is lower than the concentration of active material in known vaccines since in the known vaccines, higher concentrations were required in order to have present the required number of antigenic determinants to evoke the desired immunological response. The vaccine concentration will, of course, vary from vaccine to vaccine. Generally speaking, its concentration will be from 5 to 100 u/g, preferably 20 to 50 u/g per dose to give suitable immogenicity. It is particularly contemplated to use the vaccine in a dosage of 001 to 100, especially 01 to 10 micrograms per dose.

The vaccine will have sufficient potency in the case of a heptatitis $B_s$ vaccine to provide an anti-$HB_s$ titer of at least 1:100 when determined by passive hemaglutination (standardized by tests on a frozen anti-serum control) in at least four chimpanzees immunized with 2 doses of the standard vaccine in accordance with the recommended schedule, the anti-$HB_s$ remaining detectable at a titer of greater than 1:10 for at least a year following the onset of immunization of the chimpanzees. Naturally, the vaccine concentration can vary from these concentrations in accordance with the desired effect.

The vaccine can be administered by subcutaneous or intramuscular injection. While the preferred route would depend upon the particular vaccine, it is believed that intramuscular injection will be generally suitable. Frequency of administration will vary depending upon the vaccine and the nature and type of epitodes and their concentration in the active component. Generally speaking, the vaccine will be administered in two doses about one month apart followed by a booster at six months to one year after primary immunization. Of course, the dosage will depend upon the size of the host animal being inoculated. The subsequent doses or the booster will depend on the level of antibody in the blood as a result of the initial immunization. Licensable adjuvants conventionally employed in vaccine manufacture can be utilized.

In the case of a hepatitis vaccine as particularly contemplated herein, the same is recommended for all persons at risk of developing hepatitis B, infection and particularly those at especially high risk such as patients and staff on hemodialysis unit, medical personnel, persons of tropical populations and those

visiting the tropics. In the case of tropical populations, particularly in Africa, Asia, the Medeterranean region and South America, where high incidence of hepatitis B infections has been consistently observed, the vaccine should be administered sufficiently early in life to prevent acquisition of chronic carrier state infection which tend to occur in these regions within the first five years of life. In fact, the vaccine is useful for all persons not already protected against hepatitis B infections as a result of prior immunity.

In order to more fully illustrate the nature of the invention and the manner of practicing the same, the following examples are presented:

Example 1

A sequence of amino acids corresponding to a portion of the hepatitis B surface antigen protein was synthesized, having the following structure:

1 12
Aba Aba Thr Lys Pro Thr Asp Gly Asn Aba Thr Aba

of which the sequence Lys Pro Thr Asp Gly Asn corresponds to the H epitope, and the remaining amino acids are included to support the H epitope and to give it a proper 3-dimensional presentation for antigenicity.

Aba (amino butyric acid) residues have been included in place of cysteine residues that occur in the natural product, in order to preclude deleterious side reactions; no changes have been made in the six amino acids comprising the H epitope.

In order to assess the antigenic properties of this peptide, it was coupled to a polystyrene support (XE 305A resin beads, Rohm & Haas Co.). It was linked covalently to the beads via a two amino acid (GlyGly) bridge between the carboxyl group of the twelfth amino acid (Aba) and an amino group (benzhydrylamine) of the polystyrene resin.

This peptide bearing resin was utilized in an immunological assay using reagents available commercially as the Aistria II assay (Abbott Laboratories), a commonly used diagnostic test for hepatitis B antigen. In this case the peptidyl resin was substituted for the polystyrene beads normally used in the test, and showed a clearly positive antigenic binding activity.

The peptide has subsequently been removed from the beads by treatment with hydrofluoric acid.

The synthetic hepatitis peptide Aba Aba Thr Lys Pro Asp Gly Asn Aba Thr Aba has been shown to bind up to 9% of antibodies to the hepatitis organism, proving that it is antigenic and indicating that a valuable vaccine can be made from it.

Determination of antigenic specificities in polypeptide on polystyrene beads

To determine which antigenic specificities were present in a polypeptide prepared in accordance with this invention, the following experiment was carried out:

Monospecific antibodies to the a, d, and y specificities of HBsAg (see Prince, A. M. Brotman, B., Ikram H. in *Hepatities and Blood Transfusion* (Vyas, G. N. Perkins, H. S. and Schmid, R. editors) Grune and Stratton, New York, 1972, Pp 147—154) were prepared, titered by passive hemagglutination and diluted to a titer of 1:2 to 1:4. In addition, anti-human serum albumin was titrated against albumin coated erythrocytes, and similarly diluted with 25 mg each of uncoated polystyrene beads, normal human serum (37°C 30 min) coated beads, and beads with the attached polypeptide were washed twice with TAP buffer and then immersed in 200 ul diluted antibody. After 30 minutes at 37°C and 1 hour at 4°C, with shaking, the beads were removed by centrifugation (5000 rpm 10 min) and the antibody in the supernate was quantitated by passive hemagglutination against human type O red cells coated with HBsAg/ad by the chromic chloride method, similar cells coated with HBsAG/ay and human serum albumin and aldehyde fixed cells coated with HBsAG/ad.

The results, shown in Table A reveal that the peptide coated bead, but not the two types of control beads, adsorbed anti-a and anti-d antibodies, but not anti-y. Furthermore, none of the beads non-specifically adsorbed anti-albumin.

It was concluded that the polypeptide tested contains HBsAG/a and HBsAg/d specifications but not HBsAg/y.

| Beads | Antibody diluted to a titer of 1:2—1:4 | Titer after adsorption[1] vs. Chronic Chloride coupled HBsAg/ad coated erythrocytes | Aldehyde fixed HBsAg/ad coated erythrocytes | chronic chloride coupled HBsAg/ay coated erythrocytes | Aldehyde fixed human serum album coated erythrocytes |
|---|---|---|---|---|---|
| Control human serum coated beads | Anti HBsAg/a | 1:4 | 1:2 | 1:2 | |
| | Anti HBsAg/d | 1:4 | 1:2 | — | |
| | Anti HBsAg/y | N.D. | N.D. | N.D. | |
| | Anti human serum albumin | | | | |
| Control uncoated beads | Anti HBsAg/a | 1:2 | N.D. | N.D. | |
| | Anti HBsAg/d | 1:2 | 1:4 | — | |
| | Anti HBsAg/y | — | — | 1:4 | |
| | Anti human serum albumin | | | | 1:4 |
| Hopp polypeptide coated beads | Anti HBsAg/a | — | — | — | |
| | Anti HBsAg/d | — | — | — | |
| | Anti HBsAg/y | — | — | 1:4 | |
| | Anti human serum albumin | | | | 1:4 |
| Antibody titer before adsorption tested at the same time | Anti HBsAg/a | 1:4 | 1:2 | 1:4 | |
| | Anti HBsAg/d | 1:4 | 1:2 | — | |
| | Anti HBsAg/y | — | — | 1:4 | |
| | Anti human serum albumin | | | | 1:4 |

[1]37°C 30 min, 4°C 1 hour, with shaking. Tested at the same time as adsorbed samples.

Experiments have been carried out to demonstrate the suitability of Polyglutamic acid as a carrier for H-epitope bearing peptides. Linear poly (α) glutamic acid with an average molecular weight of 21.000 was acetylated by adding 0.1 ml of acetic anhydride to a solution of 100 mg of polyglutamic acid in 1 ml of a 50% (vol/vol) solution of water and pyridine. A ninhydrin test demonstrated that acetylation of the terminal amino group was complete after 15 minutes. This acetylation prevents further polymerization of the polyglutamic acid during subsequent steps.

The Y carboxyl groups were activated by reacting with a five-fold molar ratio of ethyl, dimethyl amino propyl carbodiimide and a five-fold ratio of N-Hydroxysuccinimide per 10 mg of acetylated polyglutamic acid dissolved in 0.5 ml of dimethyl sulfoxide. Complete esterification was achieved by 1 hr. at room temperature. The activated polyglutamic acid was precipitated and washed free of excess reagents by treating with three 5 ml washes of 0.1 N HCl. The product was dried by evaporation and stored in a freezer until needed.

H-epitope peptides may be coupled to this polymer by dissolving 10 mg of activated polyglutamic acid, and 5 mg of the peptide, in 1 ml of dimethylsulfoxide containing 1% triethylamine. Under these conditions, 5 mg of the synthetic hepatitis peptide (p. 21, line 19 of original manuscript) could be completely to 10 mg of polyglutamic acid in 15 minutes at room temperature.

Since the remaining activated carboxyls of the polyglutamic acid are not affected by the reaction, it is theoretically possible to subsequently add other amino compounds to the H peptide polymer. These compounds include other H peptides, adjuvants, and other molecules that affect the immunogenicity of the polymer.

Another modification that may affect the immunogenicity of the polymer, is to replace the N-terminal acetyl group with higher analogues, such as capryl, lauryl, or palmityl groups. These may enhance the immunogenicity directly, by acting as haptens, or may facilitate incorporation of the polymer into liposomes a procedure known to enhance immunogenicity.

It is to be understood that the term "antigen" as used herein is intended to cover exo-antigens such as bacterial, viral, parasitic and plant antigens as well as autoantigens such as those responsible for such auto-immune diseases as Myastenia Gravis and Auto Immune Thyroiditis, carditis and Nephritis.

Glossary

| Amino acid | Abbreviation |
|---|---|
| Arginine | Arg |
| Aspartic Acid | Asp |
| Glutomic Acid | Glu |
| Lysine | Lys |
| Serine | Ser |
| Asparagine | Asn |
| Glutamine | Gln |
| Glycine | Gly |
| Proline | Pro |
| Threonine | Thr |
| Alanine | Ala |
| Histidine | His |
| Cysteine | Cys |
| Methionine | Met |
| Valine | Val |
| Isoleucine | Ile |
| Leucine | Leu |
| Tyrosine | Tyr |
| Phenylalanine | Phe |
| Tryptophan | Trp |
| Alpha-Aminobutyric Acid | Aba |

**Claims**

1. A synthetic vaccine comprising a physiologically acceptable carrier on which is disposed a synthetic peptide residue containing a sequence of 6 amino acids corresponding to the sequence of such amino acids in a protein antigen or allergen where the greatest local average hydrophilicity of the antigen or allergen is found, said local hydrophilicity of said protein antigen or allergen determined by:

A. assigning relative hydrophilicity values to the amino acids of the protein antigen or allergen in accordance with the relative relationship of such amino acids as shown in the table below:

TABLE 1

| Amino acid | Hydrophilicity value |
|---|---|
| Arginine | 3.0 |
| Aspartic Acid | 3.0±1 |
| Glutamic Acid | 3.0±1 |
| Lysine | 3.0 |
| Serine | 0.3 |
| Asparagine | 0.2 |
| Glutamine | 0.2 |
| Glycine | 0.0 |
| Proline | −0.5±1 |
| Threonine | −0.4 |
| Alanine | −0.5 |
| Histidine | −0.5 |
| Cysteine | −1.0 |
| Methionine | −1.3 |
| Valine | −1.5 |
| Isoleucine | −1.81 |
| Leucine | −1.8 |
| Tyrosine | −2.3 |
| Phenylalanine | −2.5 |
| Tryptophan | −3.4 |

B. determining the repetitive local average of hydrophilicity values at a plurality of points along the amino acid chain;

C. determining from such points of repetitive averages the points of greatest local average hydrophilicity,
said vaccine when free of an amino acid sequence corresponding to the entire protein antigen or allergen evoking a protective immunological response by stimulation of antibody formation against such an antigen or allergen when introduced into a host animal.

2. A vaccine according to claim 1, wherein said vaccine comprises a plurality of epitopes.
3. A vaccine according to claim 2, wherein each epitope is part of a chain of amino acids which comprises no more than 50 amino acids.
4. A vaccine according to claim 2, wherein said chain of amino acids comprises no more than 14 amino acids.
5. A vaccine according to claim 1, wherein said amino sequence comprises the amino acid sequence of an epitope which epitope corresponds to the amino acid sequence providing greatest local average hydrophilicity of at least one of the following protein antigens: hepatitis B antigen, human histocompatability antigens, influenza hemagglutinin antigens, Fowl plague virus hemagglutinin, human chorionic gonadotropin B sub-unit, human B-2 microglobulin, human myelin basic protein, chlorera toxin beta chain, E Coli toxins, streptococcal M protein, Trypanosoma bruccei variant surface glycoprotein, herpes virus antigen, vaccina virus antigen, rabies antigen.
6. A synthetic vaccine according to claim 1, which is a vaccine for hepatitis B, said vaccine containing a peptide residue having the following sequence of amino acids: Lys Pro Thr Asp Gly Asn.

7. A synthetic vaccine according to claim 6, wherein said sequence of amino acids comprises the following sequence: Aba Aba Thr Lys Pro Thr Asp Gly Asn Aba Thr Aba.

8. A synthetic vaccine according to claim 6, wherein said sequence of amino acids comprises the following sequence: Cys Cys Thr Lys Pro Thr Asp Gly Asn Cys Thr Cys.

9. A vaccine according to claim 6, characterized by the absence of other peptide sequences of the hepatitis B virion.

10. A vaccine according to claim 9, wherein there are at least three amino acids on either side of said sequence of amino acids.

11. A vaccine according to claim 10, wherein the chain of the peptide which evokes a protective immunological response against hepatitis B does not exceed 16 amino acids.

12. A vaccine according to claim 9, wherein the chain of the peptide which evokes a protective immunological response against hepatitis B does not exceed 14 amino acids.

13. A vaccine according to claim 9, wherein each peptide chain having the said sequence of amino acids comprises between 6 and 12 amino acids in the chain.

14. A vaccine according to claim 1, wherein said carrier comprises a protein, a polysaccharide, another vaccine, an organic polymer, an organic polymer of a polypeptide.

15. A vaccine according to claim 1, comprising a residue having the following sequence of amino acids: Lys Pro Ser Asp Gly Asn.

16. A process for preparing a synthetic vaccine which comprises:

A. assigning relative hydrophilicity values to amino acids of a protein antigen or allergen, the relative hydrophilicity values being assigned in accordance with a relative relationship as shown in claim 1;

B. determining the repetitive local average of hydrophilicity values for sets of amino acids in said antigen or allergen at a plurality of points along the amino acid chain;

C. determining the sequence of 6-amino acids corresponding to the points along said protein antigen or allergen of greatest local average hydrophilicity on the basis of such averages;

D. forming a residue containing said sequence of 6 amino acids which residue is free of amino acid sequence corresponding to the entire protein antigen or allergen;

E. covently bonding said residue containing said sequence of 6 amino acids on a physiologically acceptable carrier and

F. diluting the carrier-residue of step E with a physiologically acceptable medium.

17. An immune globulin fraction comprising a monospecific antibody produced by introducing into a host animal a composition according to any of the claims 1 to 15 which provides protective immunization or decreases allergen sensitivity.

18. An immune globulin fraction according to claim 17, which is free of antibodies which do not either provide protective immunization or decrease allergen sensitivity on a host animal.

19. An immune globulin fraction according to claim 17, wherein there are a plurality of monospecific antibodies which monospecific antibodies are specific to different antigenic determinants.

20. An immune globulin fraction according to claim 17, wherein said immune globulin comprises a plurality of monospecific antibodies which are specific to different antigens or allergens.

**Revendications**

1. Vaccin de synthèse comprenant un support physiologiquement acceptable sur lequel est disposé un résidu peptide de synthèse contentant une séquence de 6 acides aminés correspondant à séquence de tels acides aminés dans un antigène ou un allergène de protéine dans laquelle se trouve l'hydrophilicité moyenne locale la plus élevée de l'antigène ou de l'allergène, ladite hydrophilité moyenne dudit antigène ou allergène de protéine étant déterminée par:

A. attribution de valeurs d'hydrophilicité relative aux acides aminés de l'antigène ou de l'allergène de protéine conformément à la relation relative de tels acides aminés comme indiqué au tableau ci-dessus:

TABLEAU 1

| Acide aminé | Valeur d'hydrophilicité |
|---|---|
| Arginine | 3,0 |
| Acide aspartique | 3,0±1 |
| Acide glutamique | 3,0±1 |
| Lysine | 3,0 |
| Sérine | 0,3 |
| Asparagine | 0,2 |
| Glutamine | 0,2 |
| Glycine | 0,0 |
| Proline | −0,5±1 |
| Thréonine | −0,4 |
| Alanine | −0.5 |
| Histidine | −0,5 |
| Cystéine | −1,0 |
| Méthionine | −1,3 |
| Valine | −1,5 |
| Isoleucine | −1,8 |
| Leucine | −1,8 |
| Tyrosine | −2,3 |
| Phénylalanine | −2,5 |
| Tryptophane | −3,4 |

B. détermination de la moyenne locale répétitive des valeurs d'hydrophilicité en une pluralité de points le long de la chaîne d'acide aminé;

C. détermination, à partir de tels points de moyennes répétitives, des points d'hydrophilicité moyenne locale la plus élevée,

ledit vaccin, lorsqu'il ne contient pas de séquence d'acides aminés correspondant à l'antigène ou à l'allergène de protéine entier, évoquant une réponse immunologique protective par stimulation de la formation d'anticorps contre un tel antigène ou allergène lorsqu'il est introduit dans un animal hôte.

2. Vaccin selon la revendication 1, dans lequel ledit vaccin comprend une pluralité d'épitopes.

3. Vaccin selon la revendication 2, dans lequel chaque épitope fait partie d'une chaîne d'acides aminés ne comprenant pas plus de 50 acides aminés.

4. Vaccin selon la revendication 3, dans lequel ladite chaîne d'acides aminés ne comprend pas plus de 14 acides aminés.

5. Vaccin selon la revendication 1, dans lequel ladite séquence d'acides aminés comprend la séquence d'acides aminés d'un épitope, lequel épitope correspond à la séquence d'acides aminés procurant l'hydrophilicité moyenne locale la plus élevée dans au moins un des antigènes de protéine suivants: antigène d'hépatite B, antigènes d'histocompatibilité humaine, antigènes d'hémagglutinine de la grippe, hémagglutinine du virus de la peste aviaire des poules, sous-unité de gonadotropine chorionique B humaine, microglobuline B-2 humaine, protéine basique de myéline humaine, chaîne beta de toxines du choléra, toxines E de Coli, protéine M streptococcale, glycoprotéine de surface s'écartant du type de trypanosome bruccei, antigène du virus de l'herpès, antigène du virus vaccinia, antigène rabies.

6. Vaccin de synthèse selon la revendication 1, qui est un vaccin pour l'hépatite B, ledit vaccin contenant un résidu peptide ayant la séquence suivante d'acides aminés: Lys Pro Thr Asp Gly Asn.

7. Vaccin de synthèse selon la revendication 6, dans lequel ladite séquence d'acides aminés comprend la séquence suivante: Aba Aba Thr Lys Pro Thr Asp Gly Asn Aba Thr Aba.

8. Vaccin de synthèse selon la revendication 6, dans lequel ladite séquence d'acides aminés comprend la séquence suivante: Cys Cys Thr Lys Pro Thr Asp Gly Asn Cys Thr Cys.

9. Vaccin selon la revendication 6, caractérisé par l'absence d'autres séquences peptide du virion hépatite B.

10. Vaccin selon la revendication 9, dans lequel existent au moins trois acides aminés de chaque côté de ladite séquence d'acides aminés.

11. Vaccin selon la revendication 10, dans lequel la chaîne du peptide qui évoque une réponse immuno-logique protective contre l'hépatite B ne dépasse pas 16 acides aminés.

12. Vaccin selon la revendication 9, dans lequel la chaîne du peptide qui évoque une réponse immuno-logique protective contre l'hépatite B ne dépasse pas 14 acides aminés.

13. Vaccin selon la revendication 9, dans lequel chaque chaîne peptide ayant ladite séquence d'acides aminés comprend entre 6 et 12 acides aminés dans chaque chaîne.

14. Vaccin selon la revendication 1, dans lequel ledit support comprend une protéine, un polysaccharide, un autre vaccin, un polymère organique, un polymère organique d'un polypeptide.

15. Vaccin selon la revendication 1, comprenant un résidu ayant la séquence suivante d'acides aminés: Lys Pro Ser Asp Gly Asn.

16. Procédé pour préparer un vaccin de synthèse qui comprend:

A. attribution de valeurs d'hydrophilicité relative à des acides aminés d'un antigène ou d'un allergène de protéine, les valeurs d'hydrophilicité relative étant attribuées conformément à une relation relative telle que représentée à la revendication 1;

B. détermination de la moyenne locale répétitive des valeurs d'hydrophilicité pour des séries d'acides aminés dans ledit antigène ou allergène en une pluralité de points le long de la chaîne d'acide aminé;

C. détermination de la séquence des 6 acides aminés correspondant aux points le long dudit antigène ou allergène de protéine ayant l'hydrophilicité moyenne locale la plus élevée sur la base de telles moyennes;

D. formation d'un résidu contenant ladite séquence de 6 acides aminés, lequel résidu ne contient pas de séquence d'acide aminé correspondant à l'antigène ou l'allergène de protéine entier;

E. liaison covalente dudit résidu contenant ladite séquence de 6 acides aminés sur un support physiologiquement acceptable, et

F. dilution du support-résidu de la phase E avec un milieu physiologiquement acceptable.

17. Fraction de globuline immune comprenant un anticorps monospécifique produit par introduction dans un animal hôte d'une composition selon l'une quelconque des revendications 1 à 15 qui procure une immunisation protective ou diminue la sensibilité allergique.

18. Fraction de globuline immune selon la revendication 17, qui ne contient pas d'anticorps qui soit ne procurent pas d'immunisation protective, soit diminuent la sensibilité allergique sur un animal hôte.

19. Fraction de globuline immune selon la revendication 17, dans laquelle existent une pluralité d'anticorps monospécifiques, lesquels anticorps monospécifiques sont spécifiques à différents déterminants antigéniques.

20. Fraction de globuline immune selon la revendication 17, dans laquelle ladite globuline immune comprend une pluralité d'anticorps monospécifiques qui sont spécifiques à différents antigènes ou allergènes.

**Patentansprüche**

1. Eine synthetische, antigene Zusammensetzung mit einem physiologisch verträglichen Träger, auf dem sich ein synthetisch erzeugter Peptidrest befindet, der eine Sequenz aus 6 Aminosäuren enthält, welche der Sequenz solcher Aminosäuren in einem Antigen- oder Allergenprotein an derjenigen Stelle entspricht, wo die Größte mittlere örtliche Hydrophilie des Antigens oder Allergens gefunden wird, wobei die örtiliche Hydrophilie dieses Antigen- oder Allergenproteins wie folgt bestimmt wurde:

A: den Aminosäuren des Antigen- oder Allergenproteins werden relative Hydrophilie-Werte entsprechend nachstehender Tabelle 1 zugeordnet:

TABELLE 1

| Aminosäure | Hydrophilie-Wert |
|---|---|
| Arginin | 3,0 |
| Asparaginsäure | 3,0±1 |
| Glutaminsäure | 3,0±1 |
| Lysin | 3,0 |
| Serin | 0,3 |
| Asparagin | 0,2 |
| Glutamin | 0,2 |
| Glycin | 0,0 |
| Prolin | −0,5±1 |
| Threonin | −0,4 |
| Alanin | −0,5 |
| Histidin | −0,5 |
| Cystein | −1,0 |
| Methionin | −1,3 |
| Valin | −1,5 |
| Isoleucin | −1,8 |
| Leucin | −1,8 |
| Tyrosin | −2,3 |
| Phenylalanin | −2,5 |
| Tryptophan | −3,4 |

B: an ainer Vielzahl von Stellen längs der Aminosäurenkette wird der sich wiederholende örtliche Mittelwert der Hydrophilie-Werte bestimmt; und

C: aus diesen Stellen der sich wiederholenden Mittelwerte werden die Stellen mit der größten mittleren örtlichen Hydrophilie bestimmt;
wobei diese antigene Zusammensetzung, sofern sie frei von einer dem gesamten Antigen- oder Allergenprotein entsprechenden Aminosäurensequenz ist, nach Einbringung in einen Wirtsorganismus eine immunologische Schutzwirkung durch Stimulierung der Antikörperbildung gegen solch ein Antigen oder Allergen hervorruft.

2. Die antigene Zusammensetzung nach Anspruch 1, wobei diese Zusammensetzung eine Anzahl Epitope aufweist.

3. Die antigene Zusammensetzung nach Anspruch 2, wobei jedes Epitop Teil einer Aminosäurenkette ist, die nicht mehr als 50 Aminosäuren aufweist.

4. Die antigene Zusammensetzung nach Anspruch 2, wobei jedes Epitop Teil einer Aminosäurenkette ist, die nicht mehr als 14 Aminosäuren aufweist.

5. Die antigene Zusammensetzung nach Anspruch 1, wobei diese Aminosäurensequenz der Aminosäurensequenz eines Epitopes entspricht, welches Epitop seinerseits diejenige Aminosäurensequenz aufweist, welche die größte mittlere örtliche Hydrophilie an wenigstens einem der nachstehenden Antigenproteine ergibt: Hepatitis B-Antigen, mit Humangewebe verträgliche Antigene, Hämagglutinin-Grippe-Antigene, Hühner- oder Geflügelpest-Virus-Hämagglutinin, Choriongonadotropin B-Untereinheit, humanes B-2-Mikroglobulin, humanes Myelin-Basisprotein, Choleratoxin-Beta-Kette, E. Coli-Toxine, Streptokokken M-Protein, das Oberflächenglycoprotein der Trypanasoma brucei-Variante (Erreger der

afrikanischen Naganahaustierseuche), Herpesvirus-Antigen, Kuhpockenvirus-Antigen, und/oder Tollwut-Antigen.

6. Die antigene Zusammensetzung nach Anspruch 1, wobei diese Zusammensetzung gegen Hepatitis B wirksam ist und einen Peptidrest enthält, der nachstehende Aminosäuresequenz aufweist: Lys Pro Thr Asp Gly Asn.

7. Die antigene Zusammensetzung nach Anspruch 6, wobei die Aminosäurensequenz die nachstehende Sequenz aufweist: Aba Aba Thr Lys Pro Thr Asp Gly Asn Aba Thr Aba.

8. Die antigene Zusammensetzung nach Anspruch 6, wobei diese Aminosäurensequenz die nachstehende Sequenz aufweist: Cys Cys Thr Lys Pro Thr Asp Gly Asn Cys Thr Cys.

9. Die antigene Zusammensetzung nach Anspruch 6, gekennzeichnet durch die Abwesenheit anderer Peptidsequenzen aus dem Hepatitis B-Virion.

10. Die antigene Zusammensetzung nach Anspruch 9, wobei wenigstens drei Aminosäuren auf jeder Seite der Aminosäurensequenz vorhanden sind.

11. Die antigene Zusammensetzung nach Anspruch 10, wobei die, eine immunologische Schutzwirkung gegen Hepatitis B hervorrufende, Peptidkette nicht mehr als 16 Aminosäuren enthält.

12. Die antigene Zusammensetzung nach Anspruch 9, wobei die, eine immunologische Schutzwirkung gegen Hepatitis B hervorrufende, Peptidkette nicht mehr als 14 Aminosäuren enthält.

13. Die antigene Zusammensetzung nach Anspruch 9, wobei jede, diese Aminosäurensequenz enthaltende Peptidkette 6 bis 12 Aminosäuren in der Kette aufweist.

14. Die antigene Zusammensetzung nach Anspruch 1, wobei der Träger ein Protein, ein Polysaccharid, eine andere Zusammensetzung, ein organisches Polymerisat oder eine organisches Polypeptid-Polymer ist.

15. Die antigene Zusammensetzung nach Anspruch 1, wobei der Peptidrest die nachstehende Aminosäurensequenz aufweist: Lys Pro Ser Asp Gly Asn.

16. Ein Verfahren zur Herstellung einer synthetischen, antigenen Zusammensetzung, gekennzeichnet durch die nachstehenden Verfahrensschritte:

A: den Aminosäuren eines Antigen- oder Allergenproteins werden relative Hydrophilie-Werte zugeordnet, wobei diese Zuordnung entsprechend den Angaben nach Tabelle 1 aus Anspruch erfolgt;

B: an einer Vielzahl von Stellen längs der Aminosäurenkette wird für Gruppen von Aminosäuren in diesem Antigen oder Allergen der sich wiederholende örtliche Mittelwert der Hydrophilie-Werte bestimmt;

C: auf der Basis dieser Mittelwerte wird die Sequenz von 6 Aminosäuren bestimmt, welche den Stellen längs dieses Antigen- oder Allergenproteins mit der größten mittleren örtlichen Hydrophilie entspricht;

D: es wird ein Peptidrest erzeugt, welcher diese Sequenz der 6 Aminosäuren enthält, wobei dieser Peptidrest frei von einer Aminosäurensequenz ist, welche dem gesamten Antigen- oder Allergenprotein entspricht;

E: dieser, die Sequenz aus 6 Aminosäuren enthaltende Peptidrest wird kovalent an einen physiologisch verträglichen Träger gebunden; und

F: das entsprechend dem Verfahrensschritt nach E erhaltene Produkt mit dem an den Träger gebundenen Peptidrest wird mit einem physiologisch verträglichen Mittel verdünnt.

17. Eine Immunglobulin-Fraktion die einen monospezifischen Antikörper aufweist, der durch Einbringung einer antigenen Zusammensetzung nach einem der Ansprüche 1 bis 15 in einen Wirtsorganismus erzeugt worden ist, welcher Antikörper eine Immunisierung hervorruft oder die Allergen-Empfindlichkeit herabsetzt.

18. Die Immunglobulin-Fraktion nach Anspruch 17, wobei diese Fraktion frei ist von irgendwelchen Antikörpern, die nicht entweder die Immunisierung oder die Herabsetzung der Allergen-Empfindlichkeit bei einem Wirtsorganismus hervorrufen.

19. Die Immunglobulin-Fraktion nach Anspruch 17, wobei diese Fraktion eine Anzahl monospezifischer Antikörper enthält, welche monospezifischen Antikörper spezifisch gegen verschiedene antigene Determinanten wirken.

20. Die Immunglobulin-Fraktion nach Anspruch 17, wobei diese Fraktion eine Vielzahl monospezifischer Antikörper enthält, welche spezifisch gegen verschiedene Antigene oder Allergene wirksame sind.